# EUROPÄISCHE PATENTANMELDUNG

(11) **EP 0 701 996 A1**
(43) Veröffentlichungstag der Anmeldung: **20.03.1996**
(21) Anmeldenummer: 95113672.0
(22) Anmeldetag: 31.08.1995
(51) Int. Cl.: C07C 209/84

(54) **Verfahren zur Abtrennung von Aminodiphenyl aus Diphenylamin**

(30) Priorität: 13.09.1994 DE 4432554; 13.09.1994 DE 4432553; 19.09.1994 DE 4433265; 25.10.1994 DE 4438003; 26.10.1994 DE 4438173
(71) Anmelder: BAYER AG, D-51368 Leverkusen (DE)
(72) Erfinder: Arndt, Frank, Dr., D-47800 Krefeld (DE); Buysch, Hans-Josef, Dr., D-47809 Krefeld (DE); Wiemers, Rudolf, Dr., D-40668 Meerbusch (DE)

(57) **Zusammenfassung**

Aminodiphenyl (ADP) kann aus Diphenylamin (DPA) dadurch abgetrennt werden, daß man das zu reinigende DPA mit einem Stoff, in dem Sauerstoff mit einer Doppelbindung an ein C-Atom, ein N-Atom oder ein weiteres O-Atom gebunden ist, bei höherer Temperatur behandelt und dann das DPA vom behandelten Gemisch abtrennt.

## Beschreibung

Die vorliegende Erfindung betrifft ein Verfahren zur Abtrennung von Aminodiphenyl (ADP) aus Diphenylamin (DPA), welches dadurch gekennzeichnet ist, daß man auf ein ADP enthaltendes DPA einen Stoff, in dem Sauerstoff mit einer Doppelbindung an ein C-Atom, ein N-Atom oder ein weiteres O-Atom gebunden ist, bei höherer Temperatur einwirken laßt und dann das DPA abtrennt.

DPA ist ein technisches Produkt, das, bedingt durch seine Herstellungsverfahren (Ullmann's Encyclopedia of Technical Chemistry, 4. Ed., Vol. 7, S. 573ff; Kirk-Othmer's Encyclopedia of Chemical Technology, 4. Ed., Vol. 2, S. 452ff) mit geringen Mengen an ADP verunreinigt ist. Die isomeren ADP, insbesondere 4-ADP, sind nachweislich cancerogen und müssen daher möglichst weitgehend aus DPA entfernt werden.

Zur Entfernung von ADP aus DPA wird in US 5.107.025 vorgeschlagen, verunreinigtes DPA in gelöster Form mit einen Ionenaustauscher zu behandeln. Diese Verfahrensweise führt zwar zum Erfolg, ist aber umständlich und aufwendig. So muß man ein vorgereinigtes, möglichst schon von groben Verunreinigungen durch Destillation befreites DPA einsetzen. Ferner benötigt man ein Lösungsmittel, das nach der Behandlung wieder entfernt werden muß. Noch weiterhin bedarf der Ionenaustauscher einer Regenerierung mit Säure, nachdem er vollständig mit dem toxischen ADP beladen ist. Dies wiederum erzeugt Probleme bei der Handhabung und Entsorgung der ADP-Ablaugen.

Es bestand daher nach wie vor ein Bedürfnis, ein einfaches und wirksames Verfahren zur Entfernung von ADP aus DPA bereitzustell:.

Überraschenderweise wurde gefunden, daß dies in hervorragender Weise gelingt, wenn man ein mit ADP verunreinigtes DPA mit einem Stoff, in dem Sauerstoff mit einer Doppelbindung an ein C-Atom, ein N-Atom oder ein weiteres O-Atom gebunden ist, bei höherer Temperatur behandelt.

Es wurde ein Verfahren zur Abtrennung von Aminodiphenyl aus Diphenylamin gefunden, das dadurch gekennzeichnet ist, daß man das zu reinigende Diphenylamin mit 1 bis 10 000 Mol eines Stoffes mit doppelt gebundenem Sauerstoff der Formel

A = O (I),

in der
- A: für den C₁-C₂₀-Rest einer Carbonylverbindung aus der Reihe der gesättigten und ungesättigten, offenkettigen oder cyclischen, aliphatischen oder aromatischen Aldehyde, Ketone und Chinone, für den C₁-C₃₀-Rest einer aliphatischen, aromatischen oder araliphatischen Mono- oder Polycarbonsäure oder eines ihrer Derivate, für den Rest eines Kohlensäurederivats, für den Rest einer Nitrosoverbindung oder für ein Sauerstoffatom steht,
pro Mol vorhandenes Aminodiphenyl bei 50 bis 310°C behandelt und dann das Diphenylamin vom behandelten Gemisch abtrennt.

Der Rest A ist damit so definiert, daß er mit dem doppelt gebundenen Sauerstoff den jeweils genannten Stoff ergibt; so erhält man aus A in der Bedeutung (CH₃)₂C mit dem doppelt gebundenen Sauerstoff Aceton, aus A in der Bedeutung HOOC-CH₂-CH-C(OH) Bernsteinsäure und aus A in der Bedeutung Cl₂C Phosgen.

Selbstverständlich können erfindungsgemäß auch Stoffe eingesetzt werden, die Stoffe der Formel (I) enthalten oder bilden.

Carbonylverbindungen A = O (I) im Sinne der vorliegenden Erfindung sind Aldehyde, Ketone und Chinone der aliphatischen und aromatischen Reihe mit 1 bis 20, bevorzugt 1 bis 15, besonders bevorzugt 1 bis 10 C-Atomen.

Eine nicht erschöpfende Aufzählung geeigneter Carbonylverbindungen ist die folgende: Benzochinon, Naphthochinon, Anthrachinon, Diphenochinon, Toluchinon, Xylochinon, Chinonmethid, Aceton, Methylethylketon, Diethylketon, Dipropylketon, Dibutylketon, Methylbutylketon, Cyclopentanon, Cyclohexanon, Methylcyclohexanon, Isopropyl-cyclohexanon, tert.-Butyl-cyclohexanon, Mesityloxid, Phoron, Isophoron, Acetylaceton, Acetessigsäureester, Acetophenon, Benzoin, Benzil, Formaldehyd, Acetaldehyd, Propionaldehyd, Butyraldehyd, Valeraldehyd, Capronaldehyd, Acrolein, Crotonaldehyd, Propiolaldehyd, Benzaldehyd, Salicylaldehyd, Tolylaldehyd, Chlorbenzaldehyd, Zimtaldehyd, Furfurol, Glykolaldehyd, Oxaldialdehyd, Glutardialdehyd, Chloracetaldehyd, Chlorpropionaldehyd. Carbonylverbindungen mit zwei oder mehr Carbonylgruppen im Molekül werden im Sinne der Erfindung als zwei oder entsprechend mehr Mol Carbonylverbindung berechnet.

Unter den aufgezählten Carbonylverbindungen sind die folgenden bevorzugt: Benzochinon, Naphthochinon, Toluchinon, Aceton, Methylethylketon, Cyclohexanon, Mesityloxid, Isophoron, Acetylaceton, Benzil, Formaldehyd, Acetaldehyd, Propionaldehyd, Acrolein, Crotonäldehyd, Benzaldehyd, Salicylaldehyd, Furfurol, Glykolaldehyd, Oxaldialdehyd, Glutardialdehyd.

Bevorzugt werden Aldehyde und Ketone, besonders bevorzugt Aldehyde, verwendet.

Geeignete Carbonsäuren der Formel A=O (I), in denen das Kohlenstoffatom, das den doppelt gebundenen Sauerstoff trägt, zusätzlich eine OH-Gruppe aufweist, sind aliphatische, aromatische und aräliphatische Mono- und Polycarbonsäuren mit 1 bis 30 C-Atomen, vorzugsweise 2 bis 20, besonders bevorzugt 2 bis 16 C-Atomen. Geeignete Derivate der Carbonsäuren sind deren Ester mit aliphatischen, aromatischen und araliphatischen Mono- und Polyhydroxyverbindungen mit 1 bis 30, vorzugsweise 1 bis 20, besonders bevorzugt 1 bis 16 C-Atomen, ferner deren Amide mit Ammoniak, primären und sekundären aromatischen Aminen mit insgesamt 6 bis 18, vorzugsweise 6 bis 12 C-Atomen, deren Halogenide, vorzugsweise Chloride, und deren Anhydride. In den Derivaten trägt in analoger Weise zu den Säuren das Kohlenstoffatom, das den doppelt gebundenen Sauerstoff trägt, zusätzlich die Säurederivat-Gruppe.

Die Carbonsäuren können durch Halogen, bevorzugt durch Chlor oder durch Hydroxy, substituiert sein und olefinische oder acetylenische Mehrfachbindungen enthalten. Ihre aromatischen Teile können Diphenylether- oder Benzophenongruppen darstellen.

Genannt seien beispielsweise Essigsäure, Propion-, Butter-, Valerian-, Capron-, Dodecan-, Pentadecan-, Stearinsäure, Benzoesäure, Tolylsäure, Chlorbenzoesäure, Salicylsäure, Ölsäure, Linolsäure, Cyclohexancarbonsäure, Maleinsäure, Bernsteinsäure, Weinsäure, Äpfelsäure, Aconitsäure, Adipinsäure, Azelainsäure, Dodecandisäure, Zitronensäure, Alkylbernsteinsäuren, Terephthalsäure, Isophthalsäure, o-Phthalsäure, Acrylsäure, Methacrylsäure, Zimtsäure, Milchsäure, Chloressigsäure, Chlorpropionsäure, Propargylsäure, Phenylpropiolsäure, Trimesinsäure, Pyromellithsäure, Diphenyletherdicarbonsäure, Diphenylethertetracarbonsäure, von Polycarbonsäuren vorzugsweise 1,2-Dicarbonsäuren; Säurechloride solcher Carbonsäuren; deren offenkettige oder cyclische Anhydride bzw. Oligo- bzw. Polyanhydride, sofern solche gebildet werden können, vorzugsweise die cyclischen Anhydride, ferner Oligomere mit Molekulargewichten bis 20.000, vorzugsweise bis 10.000 aus Acrylsäure, Methacrylsäure, Maleinsäure(anhydrid), Aconitsäure(anhydrid) bzw. deren Copolymere mit Styrol, Vinylacetat, Ethylen, Butadien und Acrylnitril. Als geeignete Hydroxyverbindungen für die Ester solcher Carbonsäuren seien genannt: Methanol, Ethanol, Butanol, Hexanol, Cyclohexanol, Hexandiol, Ethylenglykol, Propylenglykol, Glycerin, Pentaerythrit, Neopentylglykol, Phenol, Kresol, Xylenol, Hydrochinon, Resorcin, Dihydroxydiphenyl, Dihydroxydiphenylether, Bisphenol A, vorzugsweise C₁-C₄-Alkohole und Phenol. Als Amine für die Amide der genannten Carbonsäuren seien genannt: Anilin, Toluidin, Diphenylamin, Xylidin, Methyldiphenylamin und Hydrorydiphenylamin. Vorzugsweise kommt neben Ammoniak noch Diphenylamin in Frage.

In bevorzugter Weise wird ein Derivat einer Carbonsäure eingesetzt, in besonders bevorzugter Weise ein Anhydrid, ein Ester oder ein Amid, in ganz besonders bevorzugter Weise ein Anhydrid oder ein Ester bzw. Amid mit einer aromatischen Gruppe im Säure- oder im Ester- bzw. Amidteil.

Geeignete Verbindungen der Kohlensäure als Stoffe der Formel (I) sind monomere Kohlensäurederivate mit 1 bis 38 C-Atomen, vorzugsweise 1 bis 28 C-Atomen, und polymere Kohlensäurederivate mit Molgewichten bis zu 50.000, vorzugsweise 30.000, besonders bevorzugt 10.000.

Geeignet sind demnach:
- Phosgen, ihre Chlorester (II) und ihre Ester (III) mit Resten von aliphatischen, araliphatischen und aromatischen Hydroxyverbindungen R¹ und R², wobei letztere unabhänging voneinander 1 bis 20, vorzugsweise 1 bis 18, besonders bevorzugt 1 bis 15 C-Atome enthalten können:

   ClCOOR¹ (II)

   bzw.

   R¹ O-CO-OR² (III);
- nicht substituierter Harnstoff und ein- bis vierfach substituierter Harnstoff (IV), wobei die einzelnen Substituenten R³ bis R⁶ gleich oder verschieden sein können und Wasserstoff sein können oder 1 bis 20, vorzugsweise 1 bis 16, besonders bevorzugt 1 bis 12 C-Atome enthalten und aliphatischer, araliphatischer oder aromatischer Art sein können: 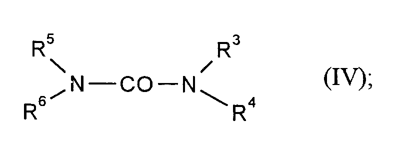
- aliphatische, araliphatische und aromatische Mono- und Polyisocyanate (V), (VI) bzw. (VII) mit aliphatischen, araliphatischen oder aromatischen Resten R⁷, R⁸ und R⁹ mit 1 bis 38, vorzugsweise 1 bis 28, besonders bevorzugt 1 bis 20 C-Atomen: 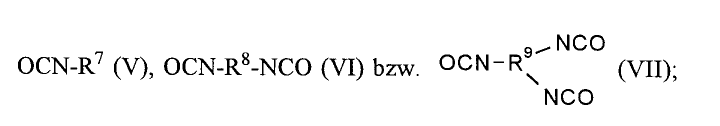
- Acylisocyanate, Chlorcarbonylisocyanate und Chlorsulfonylisocyanate;
- ein- bis dreifach mit aliphatischen, araliphatischen und aromatischen Resten R¹⁰, R¹¹ und R¹² mit 1 bis 24, bevorzugt 1 bis 18, besonders bevorzugt 1 bis 15 C-Atomen substituierte Urethane (VIII), in denen R¹¹ und R¹² unabhängig voneinander zusätzlich Wasserstoff sein können: 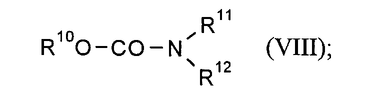
- ferner Allophanate, Isoharnstoffe, Uretdione, Uretonimine, Carbodiimide, Biurete, Polyurete, abgeleitet aus den oben angegebenen Verbindungen.

Geeignete Verbindungen der Kohlensäure sind also deren Chlorester, Monoester, Oligoester und Polyester mit aliphatischen, araliphatischen und aromatischen Mono- und Oligo-hydroxylverbindungen, welche 1 bis 20, vorzugsweise 1 bis 18, besonders bevorzugt 1 bis 15 C-Atome enthalten können. Genannt seien beispielsweise die Ester von Methanol, Ethanol, Propanol, Butanol, Benzylalkohol, Phenol und Kresol, die cyclischen Ester und Polyester von Ethylenglykol, Propylenglykol, Glycerin, Butandiol, Hexandiol, Diglykol, Triglykol, Neopentylglykol, Trimethylolpropan, Cyclohexandimethanol, Bisphenol A, Dihydroxybiphenyl, Brenzcatechin, Resorcin, Hydrochinon, Bisphenol F, Dihydroxydiphenylether und Dihydroxydiphenylsulfid.

Geeignet sind weiterhin Mono- und Oligoharnstoffe, deren Wasserstoffatome am N-Atom teilweise oder vollständig durch Alkyl-, Aralkyl- oder Arylreste substituiert sein können und pro Harnstoffgruppe 0 bis 20, vorzugsweise 0 bis 16 und besonders bevorzugt 0 bis 12 C-Atome als aliphatische, araliphatische oder aromatische Gruppen enthalten. Genannt seien beispielsweise Harnstoff selber, sodann die Harnstoffe aus Methylamin, Ethylamin, Propylamin, Butylamin, Cyclohexylamin, Benzylamin, Dimethylamin, Diethylamin, Dibutylamin, aus Anilin und Diphenylamin, die cyclischen und oligomeren Harnstoffe aus Ethylendiamin, Propylendiamin, Butylendiamin, Hexamethylendiamin und Dodecamethylendiamin.

Weiterhin geeignet sind Mono- und Oligoisocyanate mit 1 bis 38, bevorzugt 1 bis 28, besonders bevorzugt 1 bis 20 C-Atomen aliphatischer, araliphatischer und aromatischer Art pro Isocyanatgruppe. Genannt seien beispielsweise die Isocyanate aus Methyl-, Ethyl-, Propyl-, Isopropyl-, Butyl-, Amyl-, Cyclohexyl-, Lauryl-, Stearyl- und Benzylamin, aus Anilin, Toluidin, Toluylendiamin, Hexamethylendiamin, Naphthylendiamin, Isophorondiamin, Methylendianilin und dessen Oligomeren.

Geeignet sind auch Urethane aus den oben angegebenen Mono- und Oligoisocyanaten, beispielsweise mit folgenden Mono- und Oligohydroxyverbindungen: Methanol, Ethanol, Propanol, Butanol, Isopropanol, Isobutanol, Cyclohexanol, Butandiol-1,4, Ethylenglykol, Propylenglykol, Hexandiol-1,6, Neopentylglykol, Phenol, Kresol, Xylenol, tert. -Butylphenol, Chlorphenol, Hydrochinon, Brenzcatechin, Bisphenol F, Bisphenol A, Bisphenol Z, Dihydroxydiphenylsulfid, Novolake, ferner Glycerin, Trimethylolpropan, Pentaerythrit und Zucker. Bevorzugt werden die Urethane aus Monoisocyanaten mit Mono- und Oligohydroxyverbindungen, aus Monohydroxyverbindungen mit Mono- und Oligoisocyanaten und aus Diisocyanaten und Dihydroxyverbindungen.

Geeignet sind ferner Allophanate und Isoharnstoffe der Formeln 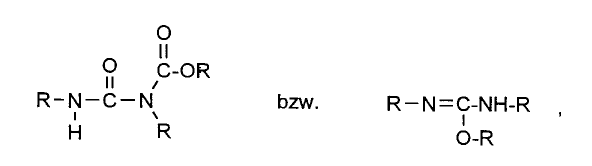
abgeleitet von den obengenannten Mono- und Oligoisocyanaten und den obengenannten Mono- und Oligohydroxyverbindungen, sodann
Uretdione 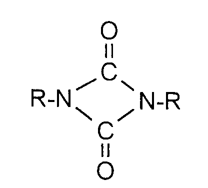
Uretonimine 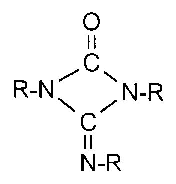
Carbodiimide R-N=C=N-R
Biurete 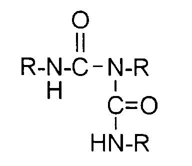
Polyurete 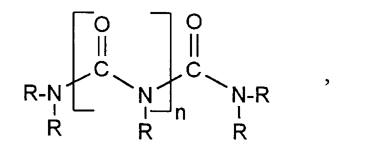
abgeleitet von oben angegebenen Mono- und Oligoisocyanaten, wobei R gleich oder verschieden und ein aliphatischer oder aromatischer C₁-C₁₂-Rest sein kann und n eine Zahl von 1-50 bedeutet.

In bevorzugter Weise werden erfindungsgemäß eingesetzt: Harnstoffe, Isocyanate, aromatische Kohlensäureester und aromatische Urethane. Besonders bevorzugt sind: Harnstoff, Diphenylcarbonat, Isocyanate auf der Basis von Anilin, Toluidin, Toluylendiamin, Methylendianilin und dessen Oligomeren und Urethane aus den genannten aromatischen Isocyanaten und Phenol.

Die oben angegebenen Verbindungen der Kohlensäure sind bekannt und beispielsweise beschrieben in Houben-Weyl, Methoden der organischen Chemie, 4. Auflage, Band E4; Kirk-Othmer, Encycl. of Chem. Techn. 3. Ed., Vol. 13, p 789 ff.; Ullmanns Encycl. of Ind. Chem. 5. Ed., Vol. A14, p 611 ff. und Vol. A5, p 197 ff..

Nitrosoverbindungen als Stoffe A=O (I) sind solche, die die Gruppe -N=O enthalten. Sie kann über ein C-Atom, ein N-Atom oder ein O-Atom mit dem Rest des Moleküles verbunden sein.

Geeignete Nitrosoverbindungen sind also aliphatische, cycloaliphatische, araliphatische, aromatische und heterocyclische Nitrosoverbindungen mit 1 bis 25, bevorzugt 1 bis 18 C-Atomen. Genannt seien beispielsweise Nitrosomethylcyclohexan, Nitroso-isobutan, Nitrosocarbonsäuren, wie α-Nitrosoisobuttersäure, α-Nitrosomalonsäurediethylester und o-Nitrosobenzoesäure, Nitrosohalogenide, wie 1-Nitroso-1-chlor-cyclohexan, 2-Nitroso-3-chlor-butan und 1-Nitroso-2-chlor-cyclohexan, Nitrosobenzol, Nitroso-phenol, Nitroso-N,N-dimethylanilin, aber auch Nitrosoheterocyclen, wie 5-Nitroso-2-amino-4-methyl-1,3-thiazol, Nitroso-antipyrin oder 4-Amino-1,3-dimethyl-5-nitroso-uracil der folgenden Formeln: 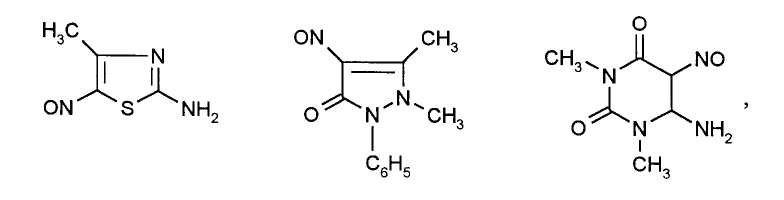
Nitrosolsäuren mit der Gruppe 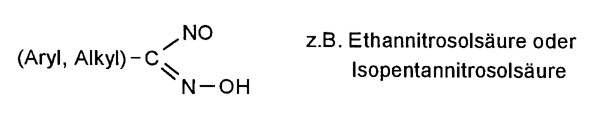
und Pseudonitrole mit der Gruppe 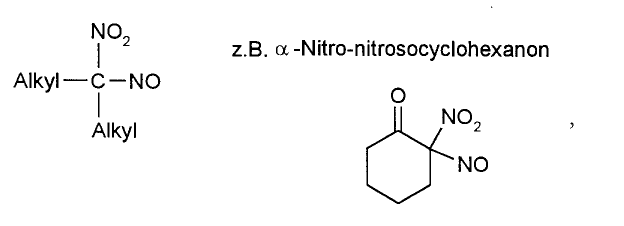
weiter Verbindungen, die die Nitrosogroppe über ein N-Atom gebunden enthalten, wie Nitrosamine, Nitrosamide, Nitrosoharnstoffe, Nitrosoguanidine, Isonitramide, Nitrosohydroxylamine. Genannt seien z.B. Dimethyl-nitrosamin, Dicyclohexylnitrosamin, Dibenzylnitrosamin, Diphenylnitrosamin, N-Nitrosomethylacetamid, N-Nitrosobutylbenzamid, N-Nitrosophenyl-isobutyramid, N-Nitroso-N-methyl-tosylamid, N-Nitroso-caprolactam, N-Nitroso-N-butyl-harnstoff, N-Nitroso-N-ethylurethan, N-Nitroso-N-methyl-guanidin und Nitrosierungsprodukte von Urotropin, wie 3,7-Dinitroso-1,3,5,7-tetraaza-bicyclo[3,3,1]nonan, 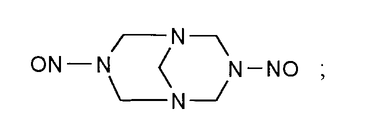
N-Nitrosophenylhydroxylamin oder beispielsweise Salze von Methylendiisonitramid 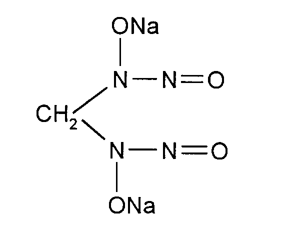
ferner Verbindungen, die die Nitrosogruppe über ein O-Atom gebunden enthalten wie salpetrige Säure und deren Ester Methylnitrit, Ethylnitrit, Isopropylnitrit und Isoamylnitrit.

Da Nitrosoverbindungen häufig in Form ihrer Dimeren vorliegen, fallen auch diese, sofern sie sich bilden können, unter die für das erfindungsgemäße Verfahren geeigneten Nitrosoverbindungen.

Schließlich sind solche Verbindungen geeignet, die organische Nitrosoverbindungen erzeugen können wie Stickoxid, NO₂, N₂O₄, N₂O₃, N₂O₅, Nitrosylchlorid und Nitrosylschwefelsäure. Schließlich kommt salpetrige Säure, beispielsweise in Form ihrer Alkalisalze, in Betracht; aus solchen Salzen kann die salpetrige Säure mit stärkeren Säuren in Freiheit gesetzt werden. Im folgenden werden solche Verbindungen ebenfalls als Nitrosoverbindungen angesprochen.

Für den Fall des Einsatzes von Sauerstoff als Stoff A = O (I) kann dieser in reiner Form, bevorzugt jedoch in Gemischen mit inerten Gasen, wie Wasserstoff, Wasserdampf, Kohlenmonoxid, Kohlendioxid, niedrigen Kohlenwasserstoffen oder Stickstoff eingesetzt werden. Besonders bevorzugt ist der Einsatz im Gemisch mit Stickstoff, ganz besonders bevorzugt der Einsatz in Form von atmosphärischer Luft.

Sauerstoff abgebende Verbindungen im erfindungsgemäßen Sinne sind solche, die Sauerstoff in peroxidischer Bindung enthalten. Genannt seien beispielsweise: Wasserstoffperoxid, beispielsweise im Gemisch mit Wasser oder organischen inerten Lösungsmitteln, wie Alkoholen; Natriumperoxid; Perborate, wie Natriumperborat und Perborax; Ketonperoxide, wie Aceton- oder Cyclohexanonperoxid; Acylperoxide, wie Peressigsäure, Perpropionsäure; Diacylperoxide, wie Dibenzoylperoxid. Weiterhin kommen Alkyl- und Aralkyl-hydroperoxide und -peroxide, wie Cyclohexylhydroperoxid, tert.-Butyl-hydroperoxid, Cumylhydroperoxid, p-Isopropylcumyl-hydroperoxid, α,α'-Di-hydroperoxi-diisopropylbenzol, Di-tert.-butylperoxid, Dicumylperoxid, tert.-Butyl-cumylperoxid, α,α'-Di-tert.-butyl-peroxi-diiso-propylbenzol in Frage. Weitere anorganische Peroxiverbindungen sind beispielsweise Peroxichromate, -niobate, -tantalate, -wolframate, -molybdate, -vanadate, -titanate, -sulfate und -phosphate. Schließlich kann Wasserstoffperoxid auch als Additionsverbindung an Harnstoff eingesetzt werden.

Die genannten Sauerstoff abgebenden Verbindungen werden in der Regel in verdünnter Form eingesetzt. Verdünnungsmittel sind beispielsweise Wasser oder inerte organische Verbindungen, beispielsweise Alkohole, wie Methanol, Isopropanol, tert.-Butanol, Ketone, wie Aceton, Methylethylketon, Methyl-isopropylketon oder Methyl-tert.-butylketon, Carbonsäuren, wie Essigsäure oder Propionsäure, Säureamide, wie Dimethylacetamid oder N-Methylpyrrolidon.

In bevorzugter Weise wird jedoch Sauerstoff, insbesondere im Gemisch mit Stickstoff eingesetzt.

Von den aufgeführten Stoffen mit doppelt gebundenem Sauerstoff der Formel A = O (I) sind die bevorzugt, in denen der Sauerstoff mit einer Doppelbindung an ein C-Atom gebunden ist. Es handelt sich bei diesen bevorzugten Stoffen demnach um solche, in den A für den C₁-C₂₀-Rest einer Carbonylverbindung aus der Reihe der gesättigten und ungesättigten, offenkettigen oder cyclischen, aliphatischen oder aromatischen Aldehyde, Ketone und Chinone, für den C₁-C₃₀-Rest einer aliphatischen, aromatischen oder araliphatischen Mono- oder Polycarbonsäure oder eines ihrer Derivaten oder für den Rest eines Kohlensäurederivats steht.

Besonders bevorzugte Stoffe sind solche, in denen A für den Rest einer der genannten Mono- oder Polycarbonsäuren oder eines ihrer Derivate, für den Rest eines Aldehyds oder für den Rest eines Kohlensäurederivats steht. In ganz besonders bevorzugten Stoffen steht A für den Rest eines Kohlensäurederivats, oder eines Aldehyds.

Zur Abtrennung des ADP ist die Behandlung mit mindestens 1 Mol Stoff der Formel A=O (I) pro Mol ADP erforderlich. Um eine möglichst vollständige Abtrennung des ADP zu erreichen, wendet man jedoch vorteilhaft einen Überschuß an Stoff der Formel (I) an, der, insbesondere zur Erfassung von Spurenmengen an ADP, sehr große Überschüsse an Stoff der Formel (I) umfaßt. Solche großen Überschüsse an Stoff der Formel (I) bei Kleinen Mengen an ADP stellen jedoch absolut gesehen immer noch eine Kleine Menge, bezogen auf die Menge des zu reinigenden DPA, dar. Die Menge an Stoff der Formel (I) beträgt demnach 1 bis 10 000 Mol, bevorzugt 5 bis 10 000 Mol, besonders bevorzugt 15 bis 7 000 Mol, ganz besonders bevorzugt 30 bis 3 000 Mol, pro Mol zu entfernendes ADP.

Die Behandlung wird bei einer Temperatur von 50 bis 310, bevorzugt 60 bis 250°C, besonders bevorzugt 60 bis 200°C, durchgeführt.

Die Behandlungszeit beträgt einige Minuten bis zu mehreren Stunden, beispielsweise 5 Minuten bis 10 Stunden, bevorzugt 1 bis 6 Stunden, und ist an sich nicht kritisch. Es läßt sich nämlich im Einzelfall leicht analytisch überprüfen, welche Zeit im Zusammenwirken mit einer gewählten Temperatur erforderlich ist, um ADP möglichst vollständig zu entfernen.

Das erfindungsgemäße Verfahren kann diskontinuierlich oder kontinuierlich durchgeführt werden. Hierzu wird beispielsweise ein Reaktionsprodukt aus der Herstellung von DPA zunächst teilweise oder vollständig destillativ von Leichtsiedern, beispielsweise von Anilin, befreit und das im Sumpf vorliegende Rohprodukt mit der nach dem Gehalt an ADP berechneten Menge an Stoff der Formel (I) im Rahmen der oben angegebenen Mengenverhältnisse versetzt und dann bei der gewählten Reaktionstemperatur in einem Behälter gerührt. Es ist jedoch auch möglich, den Stoff der Formel (I) bereits vor dem Abdestillieren der Leichtsieder, speziell des Anilins, zuzusetzen. Man kann in einer kontinuierlichen Weise auch ein vordestilliertes Rohprodukt mit dem Stoff der Formel (I) versetzen und dann kontinuierlich in einem Verweilzeitbehälter oder einem Verweilzeitrohr einer höheren Temperatur aussetzen. Niedrig siedende Stoffe der Formel (I) können dem zu reinigenden DPA in verdampfter Form zugesetzt werden; ansonsten ist der Zusatz in fester, flüssiger (aufgesschmolzener) oder in einem inerten Lösungsmittel gelöster Form möglich. Geeignete Zugabeeinrichtungen sind beispielsweise Düsen, Begasungsrührer, regulierbare Zulaufvorlagen, Pumpen und andere dem Fachmann bekannte. Ein kontinuierliches Verfahren unter Benutzung von gasförmigen oder verdampften Stoffen der Formel (I) kann in Apparaten, wie Rührkesseln, Reaktoren mit Begasungsrührern, Blasensäulen oder Rieselfilmkolonnen durchgeführt werden; der Stoff der Formel (I) wird hierbei über Düsen oder andere Verteilelemente für gasförmige Substanzen in das flüssige DPA eingebracht. Hierbei kann bei Normaldruck oder erhöhtem Druck, beispielsweise 1 bis 25 bar, bevorzugt 1 bis 15 bar, besonders bevorzugt 1 bis 10 bar gearbeitet werden. Feste oder flüssige Derivate der Kohlensäure können als solche oder in Verdünnung mit einem inerten Lösungsmittel zum DPA gegeben werden. Das behandelte Gemisch kann beispielsweise einer Kristallisation oder einer Destillation zur Gewinnung des gereinigten DPA unterworfen werden. In bevorzugter Weise wird das DPA durch Destillation vom Behandlungsgemisch abgetrennt.

Das erfindungsgemäße Verfahren kann sowohl auf ein rohes DPA in einstufiger Weise als auch auf ein vorgereinigtes DPA, gegebenenfalls in mehrstufiger Weise, angewandt werden. So kann beispielsweise rohes DPA zunächst mit einer geringeren Menge, etwa 5 bis 100 Mol Stoff der Formel (I) pro mol ADP, vorgereinigt werden und danach das vorgereinigte DPA mit einer Menge von 1000 bis 7000 Mol Stoff der Formel (I) pro Mol des weniger gewordenen restlichen ADP feingereinigt werden.

Das erfindungsgemäß erhaltene, von ADP befreite DPA ist für alle Anwendungen geeignet, beispielsweise zur Herstellung von Phenothiazinen, Kautschukstabilisatoren oder als antioxidatives Konservierungsmittel für Früchte.

### Beispiele 1 bis 9

100 g eines rohen DPA, das destillativ von Leichtsiedern, besonders Anilin (< 0,1 %), befreit war und 148 ppm 4-ADP enthielt, wurde mit einer Carbonylverbindung versetzt und eine bestimmte Zeit lang in einem Rührkolben unter Stickstoff einer Temperaturbehandlung unterzogen. Danach wurde der Kolbeninhalt bei 15 bis 20 mbar destilliert und das Destillat bezüglich ADP analysiert. Die näheren Angaben und Resultate enthält die Tabelle 1.

**Tabelle 1**

| Beispiel | Carbonylverbindung | Menge [g] | Temp. [°C] | Zeit [h] | ADP ppm |
|---|---|---|---|---|---|
| 1 | Benzaldehyd | 1 | 150 | 3 | n.n. |
| 2 | Paraformaldehyd | 1 | 150 | 3 | n.n. |
| 3 | Paraformaldehyd | 0,5 | 150 | 3 | n.n. |
| 4 | Paraformaldehyd | 0,1 | 150 | 3 | n.n. |
| 5 | Furfurol | 1 | 250 | 4 | n.n. |
| 5a | Furfurol | 0,3 | 150 | 2 | 8 |
| 6 | Mesityloxid | 1 | 200 | 4 | 10 |
| 7 | Crotonaldehyd | 1 | 180 | 4 | n.n. |
| 7a | Crotonaldehyd | 0,4 | 180 | 4 | n.n. |
| 8 | Benzil | 1 | 150 | 4 | 15 |
| 9 | Benzochinon | 1,0 | 150 | 4 | 29 |
| n.n. = nicht nachweisbar Nachweisgrenze < 5 ppm | | | | | |

### Beispiele 10 bis 12

100 g eines rohen DPA, das destillativ von Leichtsiedern, besonders Anilin (<0,1 %) befreit worden war und 85 ppm 4-ADP enthielt, wurde mit Luft in einem 250 ml Kolben unter Rühren begast. Danach wurde der Kolbeninhalt bei 15 bis 20 mbar destilliert und die im Destillat vorhandene Menge ADP bestimmt. Nähere Angaben enthält Tabelle 2.

**Tabelle 2**

| Nr. | Luftmenge | Zeit (h) | Temperatur | ADP ppm |
|---|---|---|---|---|
| 10 | 4 l/h | 3 | 250°C | <10 |
| 11 | 3 l/h | 3 | 150°C | 11 |
| 12 | 1 l/h | 4 | 200°C | <5 |

### Beispiele 13 und 14

Ersetzte man bei sonst gleicher Durchführung die Begasung mit Luft durch einen Zusatz an Peroxid, so erhielt man die in Tabelle 3 genannten Ergebnisse.

**Tabelle 3**

| Nr. | Peroxid | Zeit (h) | Temperatur | ADP ppm |
|---|---|---|---|---|
| 13 | 1 Gew.-% Benzoylperoxid | 1 h | 50°C | |
| | | + 1 h | 100°C | |
| | | + 1 h | 150°C | 18 |
| 14 | 1 Gew.-% tert.-Butylhydroperoxid | 1 h | 50°C | |
| | | + 1 h | 100°C | |
| | | + 1 h | 150°C | 13 |

### Beispiele 15 bis 17

100 g eines rohen DPA, das destillativ von Leichtsiedern, besonders Anilin (< 0,1 %), befreit war und 148 ppm 4-ADP enthielt, wurde mit einer Nitrosoverbindung in einem 250 ml-Kolben unter Rühren bei ca. 80°C vermischt und einige Stunden bei einer bestimmten Temperatur belassen. Dann wurde der Kolbeninhalt bei 15 bis 20 mbar destilliert und die im Destillat vorhandene Menge ADP bestimmt. Nähere Angaben enthält die folgende Tabelle 4.

**Tabelle 4**

| Nr. | g Nitrosoverbindung | Zeit [h] | Temp. | ADP [ppm] |
|---|---|---|---|---|
| 15 | 0,4g NaNO₂ | 4 | 250 | < 10 |
| | 0,4 g H₃PO₄ | | | |
| | 1 g H₂O | | | |
| 16 | 1 g Diphenylnitrosamin | 4 | 250 | 11 |
| 17 | 0,3 g Amylnitrit | 3 | 170 | < 10 |

### Beispiele 18 bis 23

50 g rohes, von Anilin befreites Diphenylainin und ein Kohlensäurederivat wurden in einem 250 ml-Dreihalskolben einige Stunden unter Rühren auf eine vorbestimmte Temperatur erhitzt. Danach wurde der Kolbeninhalt bei einem Druck von 10 bis 20 mbar destilliert und im Destillat der Gehalt an 4-ADP bestimmt. Das Ergebnis zeigt Tabelle 5.

**Tabelle 5**

| Nr. | Kohlensäurederivat | g | Temp. | h | ppm 4-ADP |
|---|---|---|---|---|---|
| 18 | Harnstoff | 0,4 | 180° | 4 | <5 |
| 19 | Diisocyanatodiphenylmethan | 0,5 | 160° | 3 | <5 |
| 20 | Hexamethylendiisocyanat | 0,5 | 155° | 3 | <5 |
| 21 | Hexamethylendiisocyanat | 0,3 | 140° | 2 | 14 |
| 22 | Diphenylcarbonat | 0,5 | 100° | 1 | 20 |
| | | | 150° | 2 | |
| | | | 180° | 2 | |
| 23 | Chlorkohlensäurephenylester | 0,5 | 100° | 1 | 6 |
| | | | 150° | 2 | |
| | | | 180° | 2 | |

### Beispiel 24

500 g rohes, von Anilin befreites Diphenylamin und 2,0 g Diisocyanato-diphenyl-methan (MDI) wurden 4 h unter Rühren auf 150° erhitzt und anschließend bei 10 mbar über eine Kolonne destilliert. Man erhielt ein farbloses Destillat von 478 g mit einem Gehalt an Aminodiphenyl von <5 ppm und ca. 20 g Rückstand.

### Beispiel 25

Das Beispiel 24 wurde wiederholt mit 2,0 g Harnstoff statt MDI. Das Ergebnis der Destillation glich dem von Beispiel 24. Der Gehalt an ADP lag ebenfalls bei <5 ppm.

### Beispiele 26 bis 35

100 g rohes Diphenylamin (DPA), das destillativ von Anilin (<0,1 %) befreit worden war und 125 ppm 4-Amino-diphenyl (ADP) enthielt, wurde mit einem Carbonsäurederivat im 250 ml-Kolben unter Rühren bei 80° gemischt und anschließend einige Stunden auf die vorgesehene Temperatur erhitzt. Dann wurde der Kolbeninhalt bei 15 bis 20 mbar destilliert und die im Destillat vorhandene Menge ADP bestimmt. Die Tabelle 6 enthält die Ergebnisse.

**Tabelle 6**

| Nr. | Carbonsäurederivat (g) | h | °C | ADP (ppm) |
|---|---|---|---|---|
| 26 | Phthalsäureanhydrid 0,4 | 4 | 250 | <5 |
| 27 | Phthalsäureanhydrid 0,4 | 4 | 150 | <5 |
| 28 | Pyromellithsäuredianhydrid 0,4 | 4 | 150 | <5 |
| 29 | Pyromellithsäuredianhydrid 0,4 | 4 | 180 | <5 |
| 30 | Pyromellithsäuredianhydrid 1,0 | 3 | 150 | <5 |
| 31 | Terephthalsäurediphenylester 1,0 | 4 | 180 | <5 |
| 32 | Maleinsäureanhydrid 0,5 | 3 | 160 | <5 |
| 33 | Bernsteinsäure-mono-diphenylamid 0,8 | 5 | 180 | 10 |
| 34 | N,N-Diphenylacetamid 1,0 | 6 | 150 | 9 |
| 35 | Benzoylchlorid 1,0 | 5 | 180 | <5 |

### Beispiel 36

500 g rohes, von Anilin befreites DPA enthaltend 125 ppm ADP, und 1 g Pyromellithsäuredianhydrid wurden 4 h auf 150° erhitzt; dann wurde das Gemisch bei 9 bis 10 mbar über eine Kolonne fraktioniert destilliert.

Man erhielt 8 Fraktionen an farblosem Destillat (zus. 474 g), die alle <5 ppm ADP enthielten, und 21 g Rückstand.

### Beispiel 37

Beispiel 24 wurde wiederholt, wobei jedoch das Diphenylamin noch zusätzlich 378 g Anilin enthielt. nach 4stündigem Erhitzen bei 185°C wurde das Anilin durch Vakuumdestillation abgetrennt, und danach wurde das gereinigte DPA destilliert. Alle erhaltenen Fraktionen hatten einen ADP-Gehalt von <5ppm.

### Vergleichsbeispiel

Eine Wiederholung des Beispiels 36 ohne Zusatz von Pyromellithsäuredianhydrid führte zu 8 Fraktionen mit steigenden Gehalten an ADP, beginnend bei 20 und endend bei 670 ppm ADP.

## Patentansprüche

1. Verfahren zur Abtrennung von Aminodiphenyl aus Diphenylamin, dadurch gekennzeichnet, daß man das zu reinigende Diphenylamin mit 1 bis 10 000 Mol eines Stoffes mit doppelt gebundenem Sauerstoff der Formel
A = O (I),
in der
A für den C₁-C₂₀-Rest einer Carbonylverbindung aus der Reihe der gesättigten und ungesättigten, offenkettigen oder cyclischen, aliphatischen oder aromatischen Aldehyde, Ketone und Chinone, für den C₁-C₃₀-Rest einer aliphatischen, aromatischen oder araliphatischen Mono- oder Polycarbonsäure oder eines ihrer Derivate, für den Rest eines Kohlensäurederivats, für den Rest einer Nitrosoverbindung oder für ein Sauerstoffatom steht,
pro Mol vorhandenes Aminodiphenyl bei 50 bis 310°C behandelt und dann das Diphenylamin vom behandelten Gemisch abtrennt.

2. Verfahren nach Anspruch 1, dadurch gekennzeichnet, daß im eingesetzten Stoff der Formel (I) der Sauerstoff mit einer Doppelbindung an ein C-Atom gebunden ist.

3. Verfahren nach Anspruch 2, dadurch gekennzeichnet, daß A für den C₁ - C₃₀-Rest einer Carbonsäure oder eines ihrer Derivate, für den Rest eines Aldehyds oder den Rest eines Kohlensäurederivates, bevorzugt für den Rest eines Kohlensäurederivats oder Aldehyds steht.

4. Verfahren nach Anspruch 3, dadurch gekennzeichnet, daß in Formel (I) A für den Rest einer Carbonsäure mit 2 bis 20, bevorzugt mit 2 bis 16 C-Atomen oder eines ihrer Derivate steht.

5. Verfahren nach Anspruch 4, dadurch gekennzeichnet, daß als Stoff A = O eine Carbonsäure aus der Gruppe von Essigsäure, Propion-, Butter-, Valerian-, Capron-, Dodecan-, Pentadecan-, Stearinsäure, Benzoesäure, Tolylsäure, Chlorbenzoesäure, Salicysäure, Ölsäure, Linolsäure, Cyclohexancarbonsäure, Maleinsäure, Bernsteinsäure, Weinsäure, Äpfelsäure, Aconitsäure, Adipinsäure, Azelainsäure, Dodecandisäure, Zitronensäure, Alkylbernsteinsäuren, Terephthalsäure, Isophthalsäure, o-Phthalsäure, Acrylsäure, Methacrylsäure, Zimstäure, Milchsäure, Chloressigsäure, Chlorpropionsäure, Propargylsäure, Phenylpropiolsäure, Trimesinsäure, Pyromellithsäure, Diphenyletherdicarbonsäure, Diphenylethertetracarbonsäure, von Polycarbonsäuren vorzugsweise 1,2-Dicarbonsäuren, oder eines ihrer Derivate eingesetzt wird.

6. Verfahren nach Anspruch 5, dadurch gekennzeichnet, daß als Stoff A = O ein Carbonsäurederivat, bevorzugt ein Anhydrid, ein Ester oder ein Amid, besonders bevorzugt ein Anhydrid oder ein Ester bzw. ein Amid mit einer aromatischen Gruppe im Säure- oder im Ester- bzw. Amidteil eingesetzt wird.

7. Verfahren nach Anspruch 3, dadurch gekennzeichnet, daß als Stoff A=O ein Aldehyd aus der Gruppe von Formaldehyd Acetaldehyd, Propionaldehyd, Butyraldehyd, Valeraldehyd, Capronaldehyd, Acrolein, Crotonaldehyd, Propiolaldehyd, Benzaldehyd, Salicyaldehyd, Tolylaldehyd, Chlorbenzaldehyd, Zimtaldehyd, Furfurol, Glykolaldehyd, Oxaldialdehyd, Glutardialdehyd, Chloractaldehyd, Chlorpropionaldehyd eingesetzt wird.

8. Verfahren nach Anspruch 3, dadurch gekennzeichnet, daß als Stoff A = O ein Harnstoff, ein Isocyanat, ein aromatischer Kohlensäureester oder ein aromatisches Urethan eingesetzt wird.

9. Verfahren nach Anspruch 8, dadurch gekennzeichnet, daß als Stoff A = O nicht substituierter Harnstoff, Dipehylcarbonat, ein aromatisches Isocyanat auf Basis von Anilin, Toluidin, Toluylendiamin, Methylendianilin oder dessen Oligomere und Urethane aus den genannten aromatischen Isocyanaten und Phenol eingesetzt wird.

10. Verfahren nach Anspruch 1, dadurch gekennzeichnet, daß die Behandlung bei 60 bis 250°C bevorzugt bei 60 bis 200°C, durchgeführt wird.

11. Verfahren nach Anspruch 1, dadurch gekennzeichnet, daß 5 bis 10 000 Mol, bevorzugt 15 bis 7 000 Mol, besonders bevorzugt 30 bis 3 000 Mol Carbonylverbindung pro Mol Aminodiphenyl eingesetzt werden.
